# EUROPÄISCHE PATENTSCHRIFT

(11) **EP 2 373 210 B1**
(45) Veröffentlichungstag und Bekanntmachung des Hinweises auf die Patenterteilung: **26.06.2013**
(21) Anmeldenummer: 09796972.9
(22) Anmeldetag: 23.12.2009
(51) Int. Cl.: A61B 5/00, A61M 16/01

(54) **VORRICHTUNG UND VERFAHREN ZUR ERFOLGSKONTROLLE VON SPINALANÄSTHESIEN**
DEVICE AND METHOD FOR MONITORING THE SUCCESS OF SPINAL ANESTHESIA
DISPOSITIF ET PROCEDE DESTINES A CONTROLER LA REUSSITE D'ANESTHESIES RACHIDIENNES

(30) Priorität: 03.01.2009 DE 102009003897
(43) Veröffentlichungstag der Anmeldung: 12.10.2011
(73) Patentinhaber: GP Medical Instruments GmbH, 23566 Lübeck (DE)
(72) Erfinder: GROSSMANN, Ulf, 24217 Schönberg/Kalifornien (DE)
(74) Vertreter: Hauck Patent- und Rechtsanwälte
(86) Internationale Anmeldenummer: PCT/EP2009/009238
(87) Internationale Veröffentlichungsnummer: WO 2010/075997

(56) Entgegenhaltungen:
- EP-A1- 0 236 513
- US-A- 6 002 960
- GREENE N M: "Area of differential block in spinal anesthesia with hyperbaric tetracaine" ANESTHESIOLOGY, Bd. 19, 1. Januar 1958 (1958-01-01), Seiten 45-50, XP002574069
- JETZEK-ZADER ET AL: "Increase in Skin Temperature After Spinal Anesthesia in Infants" REGIONAL ANESTHESIA AND PAIN MEDICINE, CHURCHILL LIVINGSTONE, SECAUCUS, NJ, US, Bd. 31, Nr. 6, 28. November 2006 (2006-11-28), Seiten 519-522, XP005743835 ISSN: 1098-7339
- CHAMBERLAIN D ET AL: "Changes in the skin temperature of the trunk and their relationship to sympathetic blockade during spinal anesthesia" ANESTHESIOLOGY, AMERICAN SOCIETY OF ANESTHESIOLOGISTS, PHILADELPHIA, PA, US, Bd. 65, Nr. 2, 1. August 1986 (1986-08-01) , Seiten 139-143, XP007912248 ISSN: 0003-3022
- SAITO T ET AL: "A single-injection, multi-segmental paravertebral bloc extension of somatosensory and sympathetic block in volunteers" ACTA ANAESTHESIOLOGICA SCANDINAVICA, WILEY-BLACKWELL MUNKSGAARD, DK, Bd. 45, 1. Januar 2001 (2001-01-01), Seiten 30-33, XP007912285 ISSN: 0001-5172

## Beschreibung

Gegenstand der Erfindung ist eine Vorrichtung und ein Verfahren zur Erfolgskontrolle von Spinalanästhesien in der Medizin.

Eine Spinalanästhesie ist eine rückenmarksnahe Form der Regionalanästhesie. Durch die Injektion eines Lokalanästhetikums in den Hirnwasserraum in Höhe der Lendenwirbelsäule wird die Signalübermittlung in den vom Rückenmark ausgehenden Nerven gehemmt. Dadurch wird eine zeitweilige, umkehrbare Blockade des sympathischen Nervensystems, der Sensibilität und der Motorik der unteren Körperhälfte erreicht.

Als ein Standardverfahren der Anästhesie findet die Spinalanästhesie heute Anwendung bei einer Vielzahl von Operationen am Unterbauch, im Becken, der unteren Extremität sowie in der Geburtshilfe und stellt bei diesen Eingriffen eine Alternative zu anderen Regionalverfahren wie der Periduralanästhesie und zur Vollnarkose dar.

Die Wirbelsäule des Menschen besteht aus 34 Wirbeln, die durch feste Bänder verbunden sind und das Rückmark umgeben. Zwischen den Wirbeln treten Spinalnerven aus, die den Körper segmental innervieren und so Motorik und Sensibilität ermöglichen und darüber hinaus auch Fasern des vegetativen Nervensystems (Sympathikus / Parasympathikus) fuhren. Als Bestandteil des zentralen Nervensystems ist das Rückenmark von den Hirnhäuten umgeben, die den Hirnwasserraum begrenzen, in dem das Hirnwasser zirkuliert. Dieser Liquorraum wird bei der Spinalanästhesie mit einer dünnen Kanüle punktiert. Durch die Spitze der Nadel werden Lokalanästhetika injiziert, die auf Vorder- und Hinterwurzel der Spinalnerven einwirken und deren Fähigkeit zur Übertragung von Nervenimpulsen zeitlich begrenzt aufheben.

Bekannt ist, dass bei der Spinalanästhesie unterschiedliche Qualitäten der Sensorik nacheinander ausgeschaltet werden: Zunächst wird der präganglionäre Sympathikus des vegetativen Nervensystems blockiert. Das hat eine Gefäßdilatation, ein Warmwerden der Haut und einen eventuellen Blutdruckabfall zur Folge. Danach werden Schmerz- und Temperaturfasern blockiert. Es folgen Berührung- und Druckempfinden. Zuletzt werden die Motorik, das Vibrations- und Lageempfinden ausgeschaltet.

Die Wirkhöhe der Spinalanästhesie hängt von der Ausbreitung der injizierten Wirkstoffe im Liquorraum ab, die durch Dosis und Dichte der Lokalanästhetika sowie durch die Lagerung des Patienten beeinflusst werden kann.

Bisher gibt es kein Gerät, das die Schmerzausschaltung bei dieser Form der Anästhesie anzeigen kann. Die Analgesie wurde bisher meistens mittels eines Kältesprays durch den Anästhesisten am Körper des Patienten überprüft. Hierbei musste der Patient verbal anzeigen, ob er einen entsprechenden Kältereiz noch spürt oder nicht.

Die Patentanmeldung USB6002960 offenbart eine nichtinvasive Überwachung neuraler Blockaden, die keine aktive Mitarbeit des Patienten erfordert. Die objektive Überwachung wird durch Auswertung von Elektromyogrammsignalen und Hauttemperaturmessungen ermöglicht.

Hiervon ausgehend liegt der Erfindung die Aufgabe zugrunde, eine Technik zur Verfügung zu stellen, die die Erfolgskontrolle von Spinalanästhesien erleichtert.

Die Aufgabe wird durch eine Vorrichtung mit den Merkmalen des Anspruches 1 gelöst. Vorteilhafte Ausgestaltungen der Vorrichtung sind in den Unteransprüchen 2 bis 11 angegeben.

Die erfindungsgemäße Vorrichtung zur Erfolgskontrolle von Spinalanästhesien hat
- mindestens einen elektronischen Temperatursensor zum Messen der Hautoberflächentemperatur innerhalb mindestens eines Dermatoms eines Patienten,
- eine elektronische Auswerteeinrichtung, die mit dem mindestens einen Temperatursensor verbunden ist und so ausgebildet ist, dass sie das von dem mindestens einen Temperatursensor gelieferte Messsignal darauf überwacht, ob die Hautoberflächentemperatur um etwa 2 bis 3 °C ansteigt,
- wobei die elektronische Auswerteeinrichtung ferner so ausgebildet ist, dass sie bei Ermittlung eines Anstieges der Hautoberflächentemperatur um etwa 2 bis 3 °C ermittelt, dass die Analgesie in einem Dermatom eingetreten ist, das den sakralen Lendenwirbeln um etwa 2 bis 6 Dermatome näher ist, als das Dermatom, in dem der Temperatursensor (2) die Hautoberflächentemperatur misst, und
- eine Anzeigeeinrichtung das Ergebnis der Auswertung durch die elektronische Auswerteeinrichtung optisch und/oder akustisch anzeigt.

Die erfindungsgemäße Vorrichtung geht von der Erkenntnis aus, dass bei der Sympathikolyse (Ausschaltung der sympathischen Innervierung) durch die Spinalanästhesie die "dünneren" nicht myelinisierten Nervenfasern zunächst blockiert werden und dann erst die "dickeren" myelinisierten Nervenfasern. Infolgedessen ist bei der Spinalanästhesie die Sympathikusblockade in der Regel zwei bis drei Segmente bzw. Dermatome weiter entfernt als die sensorische Blockade. Die Sympathikusblockade kann bis zu sechs Segmente bzw. Dermatome weiter entfernt als die sensorische Blockade sein. Ferner ist die sensorische Blockade etwa zwei Segmente bzw. Dermatome weiter entfernt als die motorische Blockade. Die Sympathikusblockade geht mit einer Erhöhung der Hauttemperatur um etwa 2 bis 3 °C im zugehörigen Dermatom einher. Diese Temperaturerhöhung zeigt somit an, dass eine sensorische Blockade und damit die Analgesie (Schmerzausschaltung) etwa 2 bis 3 (bis zu 6) Dermatome kaudaler eingetreten ist. Beispielsweise ist bei einer Erhöhung der Temperatur der Hautoberfläche um etwa 2 bis 3 °C im Dermatom Th2 die Analgesie etwa 2 bis 3 (-6) Dermatome kaudaler bei den Dermatomen Th4 bis Th5 (-Th8) anzunehmen. Die Erfindung macht sich diese Erkenntnis zu Nutze, indem sie aufgrund des gemessenen Temperaturanstieges von etwa 2 bis 3 °C an mindestens einem Dermatom eines Patienten ermöglicht, das 2 bis 3 (-6) Dermatome kaudaler angeordnete Dermatom als dasjenige zu identifizieren, in dem die Analgesie bereits eingetreten ist. Beispielsweise durch Anzeige des Dermatomes, in dem der Temperaturanstieg von 2 bis 3 °C eingetreten ist, und gegebenenfalls der Dermatome, in denen dieser Temperaturanstieg schon vorher eingetreten ist, wird dem Mediziner die Erfolgskontrolle der Spinalanästhesie erleichtert. Die bisherige Überprüfung mittels Kältespray und verbaler Reaktion des Patienten wird durch eine objektive Messung und Auswertung der Messergebnisse ersetzt.

Eine einfache Ausführung der erfindungsgemäßen Vorrichtung zeigt als Ergebnis der Auswertung durch die Auswerteeinrichtung das bzw. diejenigen Dermatome an, in denen der Temperaturanstieg von etwa 2 bis 3 °C eingetreten ist. Aufgrund dieser Anzeige kann angenommen werden, dass die Analgesie etwa 2 bis 3 (bis zu 6) Dermatome kaudaler eingetreten ist. Die elektronische Auswerteeinrichtung ermittelt, dass die Analgesie in einem Dermatom eingetreten ist, das den sakralen Lendenwirbeln um etwa 2 bis 6 Dermatome näher ist, als das Dermatom, in dem eine Temperaturerhöhung der Hautoberfläche von etwa 2 bis 3 °C ermittelt worden ist. Die Anzeigeeinrichtung zeigt das Dermatom, indem die Analgesie eingetreten ist, als Ergebnis der Auswertung durch die elektronische Auswerteeinrichtung an. Ggf. zeigt die Anzeigeeinrichtung zusätzlich die Dermatome an, die denen der Analgesie schon vorher eingetreten ist. Bevorzugt ermittelt die elektronische Auswerteeinrichtung, dass die Analgesie in einem Dermatom eingetreten ist, das den sakralen Lendenwirbeln um etwa 2 bis 3 Dermatome näher ist, als das Dermatom, in dem eine Temperaturerhöhung der Hautoberfläche von etwa 2 bis 3 °C ermittelt worden ist. Gemäß einer weiteren Ausgestaltung zeigt die Anzeigeeinrichtung die Dermatome, in denen die Hauptoberflächentemperatur um 2 bis 3 °C erhöht ist, und die Dermatome, in denen die Analgesie eingetreten ist, an (z.B. in verschiedenen Farben).

Der Temperatursensor kann verschieden ausgestaltet sein. Beispielsweise ist es möglich, die Hautoberflächentemperatur des Patienten großflächig mit einer Wärmekamera zu messen und die Temperaturen in den einzelnen Dermatomen mit einem automatischen Verfahren der Bildauswertung zu ermitteln. Gemäß einer anderen Ausgestaltung werden einzelne Temperatursensoren verwendet. Gemäß einer bevorzugten Ausgestaltung ist der mindestens eine Temperatursensor ein NTC-Widerstand.

Gemäß einer Ausgestaltung der Erfindung ist mindestens ein Temperatursensor über Federmittel an Mitteln zum Befestigen des Temperatursensors auf dem Körper eines Patienten gehalten. Über das Federmittel wird der Temperatursensor mit einer konstanten Andrückkraft gegen die Hautoberfläche gedrückt. Messfehler werden hierdurch vermieden.

Grundsätzlich können die Temperatursensoren einzeln auf der Hautoberfläche des Patienten fixiert werden. Gemäß einer Ausgestaltung sind mehrere Temperatursensoren in bestimmten Abständen voneinander an einem Band angeordnet, das mit Mitteln zum Befestigen am Körper eines Patienten befestigbar ist. Durch die vorgegebene Anordnung der Temperatursensoren an einem Band wird die Platzierung der Temperatursensoren am Körper des Patienten erleichtert. Bevorzugt entspricht der Abstand zweier benachbarter Temperatursensoren auf dem Band im Abstand eines oder mehrere Dermatome. Diese Ausgestaltung erleichtert die Anbringung der Temperatursensoren innerhalb verschiedener Dermatome des Patienten. Der Bemessung des Abstandes benachbarter Temperatursensoren auf dem Band kann ein Patient mittlerer Größe zugrunde gelegt werden. Ferner ist es möglich, mehrere Bänder mit Temperatursensoren in verschiedenen Abständen für Patienten verschiedener Größe bereitzustellen.

Gemäß einer Ausgestaltung ist der Temperatursensor mit einem Tape zum Befestigen am Körper eines Patienten verbunden.

Die elektronische Auswerteeinrichtung kann analog oder digital arbeiten. Es kann sich um eine programmgesteuerte elektronische Datenverarbeitungseinrichtung oder um eine reine Hardware handeln. Bevorzugt kommt eine programmierbare, digitale Auswerteeinrichtung zum Einsatz. Insbesondere kann ein PC als Auswerteeinrichtung eingesetzt werden.

Gemäß einer Ausgestaltung ist ein analoger Temperatursensor über mindestens einen Analog-Digital-Wandler mit einer digitalen Auswerteeinrichtung verbunden. Gemäß einer weiteren Ausgestaltung ist der Analog-Digital-Wandler mit einer USB-Buchse des PC's verbunden.

Die Anzeigeeinrichtung ist beispielsweise ein Bildschirm eines PC. Gemäß einer Ausgestaltung zeigt die Anzeigeeinrichtung eine Grafik eines menschlichen Körpers an, in der die Dermatome, in denen die Analgesie eingetreten ist, und/oder die Dermatome, in denen die Hautoberflächentemperatur um etwa 2 bis 3 °C angestiegen ist, grafisch hervorgehoben sind. Die grafische Hervorhebung kann z.B. durch eine sich von der übrigen Grafik abhebende Färbung der betreffenden Dermatome erfolgen.

Ferner wird die Aufgabe durch ein Verfahren mit den Merkmalen des Anspruches 12 gelöst. Vorteilhafte Ausgestaltungen des Verfahrens sind in den Unteransprüchen 13 bis 22 angegeben.

Die Erfindung wird nachfolgend anhand der anliegenden Zeichnungen eines Ausführungsbeispiels näher erläutert. In den Zeichnungen zeigen:
- Fig. 1: erfindungsgemäße Vorrichtung zur Erfolgskontrolle von Spinalanästhesien an einem Körper eines Patienten in einem grobschematischen Blockbild;
- Fig. 2: ein Abschnitt eines Bandes mit Temperatursensoren in der Draufsicht;
- Fig. 3: ein vergrößertes Detail des Bandes von Fig. 2 in einem Vertikalschnitt.

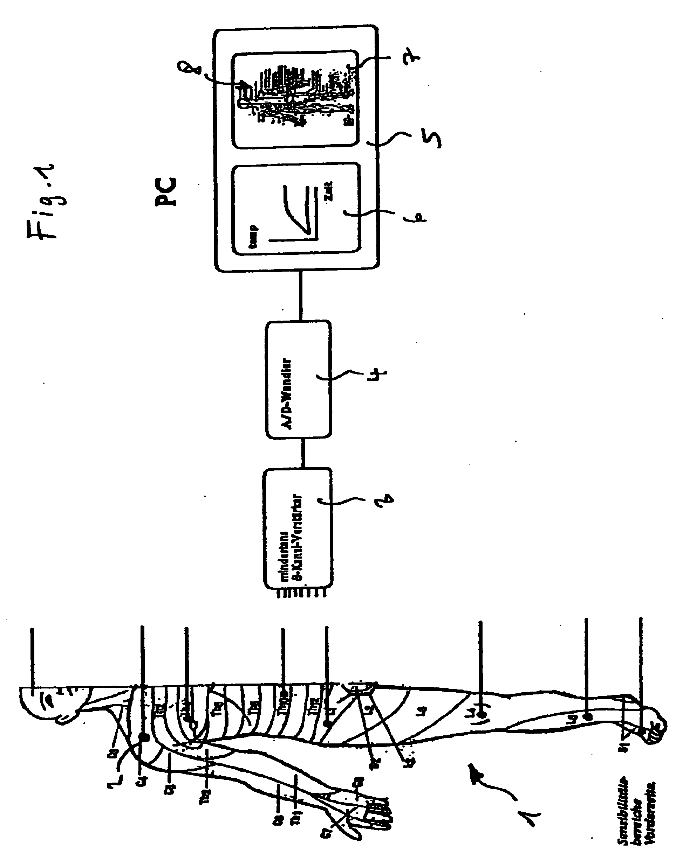

Gemäß Fig. 1 ist die Hautoberfläche eines Patienten 1 in verschiedene Dermatome segmentiert, die mit Bezeichnungen wie z. Bsp. Th2, L3 und C4 bezeichnet sind. Auf bestimmten Dermatomen sind Temperatursensoren 2 angebracht. Die Temperatursensoren 2 sind über einen Verstärker 3 mit mindestens 8 Kanälen mit einem Analog-Digital-Wandler 4 verbunden. Der Analog-Digital-Wandler 4 tastet die Ausgangskanäle des Verstärkers 3 ab und wandelt das verstärkte, analoge Messsignal in ein digitales Signal um.

Der Analog-Digital-Wandler 4 ist an einen PC 5 angeschlossen. Der PC 5 ermittelt, ob die von den Temperatursensoren 2 gemessene Hauttemperatur um etwa 2 bis 3 °C ansteigt, wie im Temperatur-Zeit-Diagramm 6 gezeigt. Wenn der PC 5 einen Anstieg um 2 bis 3 °C feststellt, errechnet er, dass eine Analgesie in einem etwa 2 bis 3 (-6) Dermatome kaudaler angeordneten Dermatom eingetreten ist. Die Dermatome, in denen die Analgesie festgestellt wurde, werden auf einem Bildschirm 7 mit einer Grafik 8 eines menschlichen Körpers dargestellt.

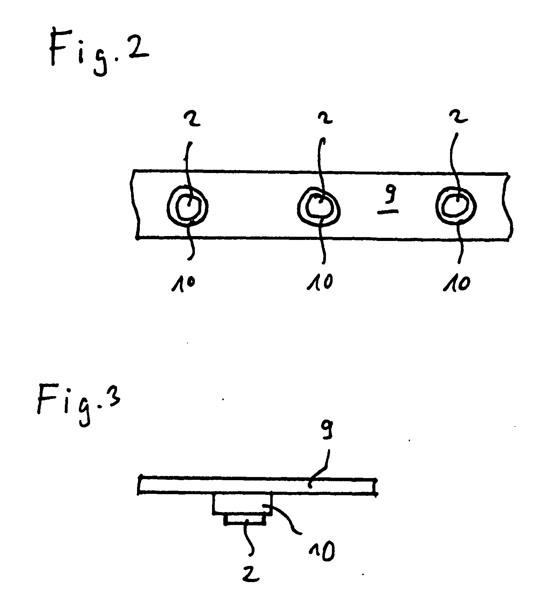

Gemäß Fig. 2 und 3 sind mehrere Temperatursensoren 2 auf einem Band 9 jeweils unter Zwischenlage eines Schaumstoffpolsters 10 befestigt. Die Abstände zwischen benachbarten Temperatursensoren 2 entsprechen den Abständen zwischen bestimmten Dermatomen. Das Band 9 ist mittels Tapes, die quer auf dem Band 9 angebracht werden, am Körper 1 befestigbar.

## Patentansprüche

1. Vorrichtung zur Erfolgskontrolle von Spinalanästhesien mit
- mindestens einem elektronischen Temperatursensor (2) zum Messen der Hautoberflächentemperatur innerhalb mindestens eines Dermatoms eines Patienten,
- einer elektronischen Auswerteeinrichtung (5), die mit dem mindestens einem Temperatursensor (2) verbunden ist und so ausgebildet ist, dass sie das von dem mindestens einen Temperatursensor (2) gelieferte Messsignal darauf überwacht, ob die Hautoberflächentemperatur um etwa 2 bis 3 °C ansteigt,
- wobei die elektronische Auswerteeinrichtung (5) ferner so ausgebildet ist, dass sie bei Ermittlung eines Anstieges der Hautoberflächentemperatur um etwa 2 bis 3 °C ermittelt, dass die Analgesie in einem Dermatom eingetreten ist, das den sakralen Lendenwirbeln um etwa 2 bis 6 Dermatome näher ist, als das Dermatom, in dem der Temperatursensor (2) die Hautoberflächentemperatur misst, und
- eine Anzeigeeinrichtung (7), die das Ergebnis der Auswertung durch die elektronische Auswerteeinrichtung optisch und/oder akustisch anzeigt.

2. Vorrichtung nach Anspruch 1, bei der die elektronische Auswerteeinrichtung (5) so ausgebildet ist, dass sie bei Ermittlung eines Anstieges der Hautoberflächen-temperatur um etwa 2 bis 3 °C ermittelt, dass die Analgesie in einem Dermatom eingetreten ist, das den sakralen Lendenwirbeln um etwa 2 bis 3 Dermatome näher ist, als das Dermatom, in dem der Temperatursensor (2) die Hautober-flächentemperatur misst.

3. Vorrichtung nach Anspruche 1 oder 2, bei der der mindestens eine Temperatursensor (2) ein NTC-Widerstand ist.

4. Vorrichtung nach einem der Ansprüche 1 bis 2, bei der mindestens ein Temperatursensor (2) über Federmittel an Mitteln zum Befestigen (9) auf dem Körper eines Patienten gehalten ist.

5. Vorrichtung nach einem der Ansprüche 1 bis 4, bei der mehrere Temperatursensoren (2) in bestimmten Abständen voneinander an einem Band (9) angeordnet ist, das mit Mitteln zum Befestigen am Körper eines Patienten befestigbar ist.

6. Vorrichtung nach einem der Ansprüche 1 bis 5, bei der mindestens ein Temperatursensor mit einem Tape zum Befestigen am Körper eines Patienten verbunden ist.

7. Vorrichtung nach Anspruch 5 oder 6, bei der der Abstand zweier benachbarter Temperatursensoren (2) auf dem Band (9) den Abstand eines oder mehrerer Dermatome entspricht.

8. Vorrichtung nach einem der Ansprüche 1 bis 7, bei der der mindestens eine Temperatursensor (2) über mindestens einen Analog-Digital-Wandler (4) mit einer digitalen Auswerteeinrichtung (5) verbunden ist.

9. Vorrichtung nach Anspruch 8, bei der die digitale Auswerteeinrichtung (5) ein PC ist.

10. Vorrichtung nach Anspruch 8 und 9, bei der der Analog-Digital-Wandler (4) mit einer USB-Buchse des PC's (5) verbunden ist.

11. Vorrichtung nach einem der Ansprüche 1 bis 10, bei der die Anzeigeeinrichtung (7) eine Grafik (8) eines menschlichen Körpers anzeigt, in der die Dermatome, in denen die Analgesi eingetreten sind, und/oder die Dermatome, in denen die Hautoberflächentemperatur um etwa 2 bis 3 °C angestiegen ist, grafisch hervorgehoben sind.

12. Verfahren zur Erfolgskontrolle von Spinalanästhesien, bei dem
- mindestens ein elektronischer Temperatursensor die Hautoberflächentemperatur innerhalb mindestens eines Dermatoms eines Patienten misst,
- eine elektronische Auswerteeinrichtung das von den mindestens einen Temperatursensor gelieferte Messsignal auf einen Anstieg der Hautoberflächentemperatur um etwa 2 bis 3 °C überwacht,
- die elektrische Auswerteeinrichtung bei einem Anstieg der Hautoberflächentemperatur um etwa 2 bis 3 °C die Analgesie in einem den sakralen Lendenwirbeln des Patienten um 2 bis 6 Dermatome als der Temperatursensor näheren Dermatom ermittelt und
- eine Anzeigeeinrichtung das Ergebnis der Auswertung durch die elektronische Auswerteeinrichtung anzeigt.

13. Verfahren nach Anspruch 12, bei dem die Anzeigeeinrichtung die Analgesie in dem ermittelten Dermatom anzeigt.

14. Verfahren nach Anspruch 12 oder 13, bei dem die elektronische Auswerteeinrichtung bei einem Anstieg der Hautoberflächentemperatur um etwa 2 bis 3 °C die Analgesie in einem den sakralen Lendenwirbeln des Patienten um 2 bis 3 Dermatome als der Temperatursensor näheren Dermatom ermittelt und die Anzeigeeinrichtung die Analgesie in dem ermittelten Dermatom anzeigt.

15. Verfahren nach einem der Ansprüche 12 bis 14, bei dem mindestens ein Temperatursensor die Hautoberfläche innerhalb verschiedener Dermatome misst.

16. Verfahren nach Anspruch 15, bei dem mindestens ein Temperatursensor auf der Hautoberfläche angeordnet ist.

17. Verfahren nach Anspruch 16, bei dem mehrere Temperatursensoren in einem Abstand von mindestens einem Dermatom auf der Hautoberfläche des Patienten angeordnet sind.

18. Verfahren nach Anspruch 16 oder 18, bei dem mindestens einen Sensor über ein Federmittel gegen die Hautoberfläche gedrückt wird.

19. Verfahren nach einem der Ansprüche 16 bis 18, bei dem Temperatursensoren mittels eines Bandes, auf dem sie in bestimmten Abständen voneinander angeordnet sind, auf der Hautoberfläche des Patienten platziert werden.

20. Verfahren nach einem der Ansprüche 12 bis 11, bei dem die Temperatursensoren ein analoges Messsignal liefern, das mittels des Analog-Digital-Wandlers in ein digitales Signal umgewandelt wird das digital von der Auswerteeinrichtung verarbeitet wird.

21. Verfahren nach einem der Ansprüche 12 bis 20, bei dem die Anzeigeeinrichtung in einer Grafik eines menschlichen Körpers die Dermatome, in denen die Analgesie eingetreten ist, und/oder die Dermatome, in denen die Hautoberfläche um mindestens etwa 2 bis 3 °C angestiegen ist, grafisch hervorgehoben anzeigt.

22. Verfahren nach einem der Ansprüche 12 bis 21, bei dem die Auswerteinrichtung das Ergebnis der Auswertung optisch und/oder akustisch anzeigt.

## Claims

1. A device for the monitoring of the success of spinal anesthesia with
• at least one electronic temperature sensor (2) for measuring the skin surface temperature within at least one dermatome of a patient,
• an electronic evaluation device (5), which is connected with the at least one temperature sensor (2) and is designed such that it monitors the measurement signal delivered by the at least one temperature sensor (2) to determine whether the skin surface temperature increases by approximately 2 to 3°C,
• wherein the electronic evaluation device (5) is also designed such that in the case of the determination of an increase in the skin surface temperature by approx. 2 to 3° C it determines that the analgesia has occurred in a dermatome, which is closer to the sacral lumbar vertebrae by approximately 2 to 6 dermatomes than the dermatome, in which the temperature sensor (2) measures the skin surface temperature, and
• a display device (7) that displays the result of the evaluation by the electronic evaluation device optically and/or acoustically.

2. The device according to claim 1, in which the electronic evaluation unit (5) is designed such that in the case of the determination of an increase in the skin surface temperature by approx. 2 to 3° C it determines that the analgesia has occurred in a dermatome, which is closer to the sacral lumbar vertebrae by approximately 2 to 3 dermatomes than the dermatome, in which the temperature sensor (2) measures the skin surface temperature.

3. The device according to claim 1 or 2, in which the at least one temperature sensor (2) is an NTC resistor.

4. The device according to one of claims 1 through 2, in which at least one temperature sensor (2) is held via spring means on means for fastening (9) on the body of a patient.

5. The device according to one of claims 1 through 4, in which several temperature sensors (2) are arranged on a band (9) at certain distances from each other, which is fastenable with means for fastening on the body of a patient.

6. The device according to one of claims 1 through 5, in which the at least one temperature sensor is connected with a tape for fastening on the body of a patient.

7. The device according to claim 5 or 6, in which the distances between two neighboring temperature sensors (2) on the band (9) corresponds with the distances between one or more dermatomes.

8. The device according to one of claims 1 through 7, in which the at least one temperature sensor (2) is connected with a digital evaluation device (5) via at least one analog-digital converter (4).

9. The device according to claim 8, in which the digital evaluation device (5) is a PC.

10. The device according to claim 8 or 9, in which the analog-digital converter (4) is connected with a USB port of the PC (5).

11. The device according to one of claims 1 through 10, in which the display device (7) shows a graphic (8) of a human body, in which the dermatomes, in which the analgesia have occurred, and/or the dermatomes, in which the skin surface temperature has increased by approximately 2 to 3° C, are highlighted graphically.

12. The method for monitoring the success of spinal anesthesia, in which
• at least one electronic temperature sensor measures the skin surface temperature within at least one dermatome of a patient,
• an electronic evaluation device monitors the measurement signal delivered by the at least one temperature sensor for an increase in the skin surface temperature by approximately 2 to 3° C,
• the electronic evaluation device in the case of an increase in the skin surface temperature by approximately 2 to 3° C determines the analgesia in a dermatome closer to the sacral lumbar vertebrae of the patient by 2 to 6 dermatomes than the temperature sensor and
• a display device shows the result of the evaluation by the electronic evaluation device.

13. The method according to claim 12, in which the display device display the analgesia in the determined dermatome.

14. The method according to claim 12 or 13, in which the electronic evaluation device determines, in the case of an increase in the skin surface temperature by approximately 2 to 3° C, the analgesia in a dermatome closer to the sacral lumbar vertebrae of the patient by 2 to 3 dermatomes than the temperature sensor and the display device displays the analgesia in the determined dermatome.

15. The method according to one of claims 12 through 14, in which at least one temperature sensor measures the skin surface within different dermatomes.

16. Method according to claim 15, in which at least one temperature sensor is arranged on the skin surface.

17. The method according to claim 16, in which several temperature sensors are arranged on the skin surface of the patient at a distance of at least one dermatome.

18. The method according to claim 16 or 18, in which at least one sensor is pressed against the skin surface via a spring means.

19. The method according to one of the claims 16 through 18, in which the temperature sensors are placed on the skin surface of the patient by means of a band, on which they are arranged at certain distances from each other.

20. The method according to one of the claims 12 through 9, in which the temperature sensors deliver an analog measurement signal, which is converted into a digital signal by means of the analog-digital converter and which is digitally processed by the evaluation device.

21. The method according to one of claims 12 through 20, in which the display device displays in a graphically highlighted manner in a graphic of a human body the dermatomes, in which the analgesia has occurred, and/or the dermatomes, in which the skin surface has increased by approximately 2 to 3° C.

22. The method according to one of claims 12 through 21, in which the evaluation device displays the result of the evaluation optically and/or acoustically.

## Revendications

1. Dispositif destiné à contrôler la réussite d'anesthésies rachidiennes, avec
• au moins un capteur de température électronique (2) pour mesurer la température de surface de la peau dans au moins un dermatome d'un patient,
• un dispositif d'évaluation électronique (5) qui est relié à l'au moins un capteur de température (2) et qui est conçu de manière à surveiller le signal de mesure fourni par l'au moins un capteur de température (2) pour voir si la température de surface de la peau augmente d'environ 2 à 3 °C,
• le dispositif d'évaluation électronique (5) étant en outre conçu de manière qu'en cas de la découverte d'une montée de la température de surface de la peau d'environ 2 à 3 °C, il constate que l'analgésie est achevée dans un dermatome qui est plus proche aux vertèbres lombaires sacrés pour environ 2 à 6 dermatomes que le dermatome dans lequel le capteur de température (2) mesure la température de surface de la peau, et
• un dispositif d'affichage (7) qui affiche visuellement et/ou acoustiquement le résultat de l'évaluation effectuée par le dispositif d'évaluation électronique.

2. Dispositif selon la revendication 1, dans lequel le dispositif d'évaluation électronique (5) est conçu de manière qu'en cas de la découverte d'une montée de la température de surface de la peau d'environ 2 à 3 °C, il constate que l'analgésie est achevée dans un dermatome qui est plus proche aux vertèbres lombaires sacrés pour environ 2 à 3 dermatomes que le dermatome dans lequel le capteur de température (2) mesure la température de surface de la peau

3. Dispositif selon la revendication 1 ou 2, dans lequel l'au moins un capteur de température (2) est une résistance NTC.

4. Dispositif selon l'une quelconque des revendications 1 à 2, dans lequel au moins un capteur de température (2) est pris dans des moyens d'attache (9) sur le corps du patient à travers un ensemble de ressorts.

5. Dispositif selon l'une quelconque des revendications 1 à 4, dans lequel plusieurs capteurs de température (2) sont disposés à certains écarts de l'un à l'autre sur un cordon (9) qui se laisse attacher sur le corps d'un patient à l'aide de moyens d'attache.

6. Dispositif selon l'une quelconque des revendications 1 à 5, dans lequel au moins un capteur de température (2) est relié à un ruban destiné à la fixation sur le corps d'un patient

7. Dispositif selon la revendication 5 ou 6, dans lequel la distance de deux capteurs de température (2) avoisinants sur le cordon (9) correspond à la distance d'un ou plusieurs dermatomes.

8. Dispositif selon l'une quelconque des revendications 1 à 7, dans lequel l'au moins un capteur de température (2) est relié à un dispositif d'évaluation (5) numérique à travers un convertisseur numérique-analogique (4).

9. Dispositif selon la revendication 8, dans lequel le dispositif d'évaluation (5) numérique est un ordinateur personnel.

10. Dispositif selon la revendication 8 et 9, dans lequel le convertisseur numérique-analogique (4) est relié à une douille USB de l'ordinateur personnel (5).

11. Dispositif selon l'une quelconque des revendications 1 à 10, dans lequel le dispositif d'affichage (7) affiche une représentation graphique d'un corps humain, dans laquelle des dermatomes dans lesquels l'analgésie est achevée et/ou les dermatomes dans lesquels la température de surface de la peau est augmentée d'environ 2 à 3 °C, sont graphiquement accentués.

12. Procédé destiné à contrôler la réussite d'anesthésies rachidiennes, dans lequel
• au moins un capteur de température électronique mesure la température de surface de la peau dans au moins un dermatome d'un patient,
• un dispositif d'évaluation électronique surveille le signal de mesure fourni par l'au moins un capteur de température pour voir si la température de surface de la peau augmente d'environ 2 à 3 °C,
• le dispositif d'évaluation électrique constate, en cas d'une montée de la température de surface de la peau d'environ 2 à 3 °C, l'analgésie dans un dermatome qui est plus proche aux vertèbres lombaires sacrés que le capteur de température (2) pour environ 2 à 6 dermatomes,
• un dispositif d'affichage affiche le résultat de l'évaluation effectuée par le dispositif d'évaluation électronique.

13. Procédé selon la revendication 12, dans lequel le dispositif d'affichage affiche l'analgésie dans le dermatome constaté.

14. Procédé selon la revendication 12 ou 13, dans lequel le dispositif d'évaluation électronique constate, en cas d'une montée de la température de surface de la peau d'environ 2 à 3 °C, l'analgésie dans un dermatome qui est plus proche aux vertèbres lombaires sacrés que le capteur de température (2) pour environ 2 à 6 dermatomes, et le dispositif d'affichage affiche l'analgésie dans le dermatome constaté.

15. Procédé selon l'une quelconque des revendications 12 à 14, dans lequel au moins un capteur de température mesure la surface de la peau dans divers dermatomes.

16. Procédé selon la revendication 15, dans lequel au moins un capteur de température est arrangé sur la surface de la peau.

17. Procédé selon la revendication 16, dans lequel plusieurs capteurs de température sont disposés sur la surface de la peau d'un patient en un écart d'au moins un dermatome.

18. Procédé selon la revendication 16 ou 18, dans lequel au moins un capteur est pressé contre la peau à travers un ensemble de ressorts.

19. Procédé selon l'une quelconque des revendications 16 à 18, dans lequel des capteurs de température sont placés sur la surface de la peau d'un patient à l'aide d'un cordon sur lequel ils sont disposés en certains écarts de l'un à l'autre.

20. Procédé selon l'une quelconque des revendications 12 à 19, dans lequel les capteurs de température fournissent un signal de mesure analogique, qui est converti en un signal numérique moyennant le convertisseur numérique-analogique et qui est numériquement traité par le dispositif d'évaluation.

21. Procédé selon l'une quelconque des revendications 12 à 20, dans lequel le dispositif d'affichage affiche graphiquement accentués dans une représentation graphique d'un corps humain ces dermatomes dans lesquels l'analgésie est achevée et/ou les dermatomes dans lesquels la température de surface de la peau est augmentée d'environ 2 à 3 °C.

22. Procédé selon l'une quelconque des revendications 12 à 21, dans lequel le dispositif d'affichage affiche visuellement et/ou acoustiquement le résultat de l'évaluation.
